# EUROPEAN PATENT APPLICATION

(11) **EP 2 868 261 A1**
(43) Date of publication of application: **06.05.2015**
(21) Application number: 13812712.1
(22) Date of filing: 17.06.2013
(51) Int. Cl.: A61B 5/00

(54) **ICTERUS METER AND ICTERUS METER OUTPUT METHOD**

(30) Priority: 02.07.2012 JP 2012148418
(71) Applicant: Konica Minolta, Inc., Tokyo 100-7015 (JP)
(72) Inventor: YASHIRO, Takehiro, Tokyo 100-7015 (JP); TATEDA, Norihiro, Tokyo 100-7015 (JP)
(74) Representative: Hoffmann Eitle
(86) International application number: PCT/JP2013/003772
(87) International publication number: WO 2014/006826

(57) **Abstract**

Disclosed are a jaundice meter and an output method for the jaundice meter, wherein input therapeutic information regarding a medical treatment and a measured degree of jaundice are stored in the storage unit in mutually associate relation, and, when outputting the degree of jaundice as a measurement result, the therapeutic information stored in the storage unit can be output from the output unit, together with the degree of jaundice.

## Description

### Technical Field

The present invention relates to a jaundice meter and an output method for the jaundice meter, and more particularly to a jaundice meter capable of outputting therapeutic information regarding a medical treatment for a measurement target, and an output method for the jaundice meter.

### Background Art

Jaundice is a symptom emerging as a result of a phenomenon that, in a condition where there is a high level of bilirubin in blood, i.e., hyperbilirubinemia, caused when bilirubin generated from breakdown of hemoglobin in red blood cells is not excreted from a living body due to some kind of disorder, the high-level bilirubin in the blood is deposited in a living tissue, thereby causing yellowish pigmentation of the living tissue. Bilirubin has a high affinity for fibroelastic tissue, so that it is deposited in a skin, a sclera, a blood vessel and the like abundant in fibroelastic tissue. A fetus in a prenatal period has a red blood cell count about 1.5 to 2 times greater than that of an adult human, in order to compensate for deficiency of oxygen due to poor efficiency of oxygen exchange by placenta, as compared to oxygen exchange by lungs. When lung breathing becomes possible after birth, the number of red blood cells in blood becomes excess, and thus an excess part of the red blood cells are broken down. As a result of the above physiological phenomenon, a newborn infant becomes jaundiced. This jaundice is referred to as "neonatal jaundice".

In some cases, the neonatal jaundice becomes serious disease, and involves a risk of exerting a critical influence on life and brain development of a newborn infant. For this reason, it is necessary to early and appropriately detect and treat the neonatal jaundice. From the standpoint of accurate determination on an intensity of jaundice symptoms (degree (of severity) of jaundice), a bilirubin level in blood should be measured. However, it is difficult, and not realistic, to collect blood from each newborn infant to measure a bilirubin level in the blood.

Therefore, a jaundice meter for measuring a degree of jaundice has heretofore been developed, and an example thereof is disclosed in the following Patent Literatures 1 and 2. Each jaundice meter disclosed in the Patent Literatures 1 and 2 is an apparatus designed to measure a degree of jaundice based on a phenomenon that a light component on a short wavelength side is absorbed by bilirubin deposited in a subcutaneous tissue in a greater amount as compared to a light component on a long wavelength side. More specifically, in each jaundice meter disclosed in these Patent Literatures, the Lambert-Beer law is utilized, wherein, first of all, light is emitted from the jaundice meter to enter a skin. The entering light is scattered by a fatty layer or the like of the subcutaneous tissue having bilirubin deposited therein, and the resulting backwardly scattered light is emitted from a surface of the skin and received by the jaundice meter. The jaundice meter is operable, based on the received backwardly scattered light, to derive two light intensities at a first wavelength having a relatively large bilirubin absorption coefficient and a second wavelength having a relatively small bilirubin absorption coefficient, and a concentration of the bilirubin deposited in the subcutaneous tissue is measured from a difference between the light intensities. Such an optical jaundice meter has an advantage of being able to obtain a measurement result in a non-invasive manner and within a relatively short period of time.

Meanwhile, in a situation where jaundice emerges and it is determined that a medical treatment is required, as a therapeutic method therefor, a medical treatment, such as phototherapy designed to emit a light beam to a patient so as to break down bilirubin in blood to allow the bilirubin to become more soluble in water, is performed.

The above optical jaundice meter is configured to estimate a bilirubin concentration in blood by measuring a concentration of bilirubin deposited in a subcutaneous tissue, i.e., to derive a bilirubin concentration in blood, in an indirect manner. Thus, a relationship between a bilirubin concentration in blood, and a result of measurement using the optical jaundice meter becomes different depending on the presence or absence of a medical treatment. Therefore, it is necessary to distinguish the result of measurement using the optical jaundice meter, according to the presence or absence of a medical treatment. Heretofore, the presence or absence of a medical treatment has been determined by checking a medical treatment record or a record described for each patient. This is disadvantageously time-consuming and cumbersome.

The jaundice meter disclosed in the Patent Literature 1 has first and second display modes. The first display mode is configured to directly display an optical concentration difference corresponding value calculated from respective reflectances of two light components of first and second wavelengths, and the second display mode is configured to convert the optical concentration difference corresponding value to a bilirubin concentration in serum, and display the converted value. The Patent Literature 1 neither discloses nor suggests displaying the presence or absence of a medical treatment.

### Citation List

### Patent Literature

Patent Literature 1: JP 4-127035 A (JP 2943294 B)
Patent Literature 2: JP 2000-279398 A

### Summary of Invention

The present invention has been made in view of the above circumstances, and an object thereof is to provide a jaundice meter capable of outputting therapeutic information regarding a medical treatment for a measurement target, and an output method for the jaundice meter.

In a jaundice meter and an output method for the jaundice meter, according to the present invention, therapeutic information regarding a medical treatment is stored in a storage unit, and, when a degree of jaundice as a measurement result is output, the therapeutic information is output from an output unit together with the degree of jaundice

These and other objects, features, and advantages of the present invention will become apparent upon reading of the following detailed description along with the accompanying drawings.

### Brief Description of Drawings

FIG. 1 is six orthogonal views illustrating an external configuration of a jaundice meter according to a first embodiment of the present invention.
FIG. 2 is perspective views illustrating the external configuration of the jaundice meter according to the first embodiment.
FIG. 3 is a block diagram illustrating an electrical configuration of the jaundice meter according to the embodiment.
FIG. 4 is an explanatory diagram of measurement target information stored in a measurement target information storage section of the jaundice meter according to the first embodiment.
FIG. 5 is a flowchart illustrating an operation of the jaundice meter according to the first embodiment.
FIG. 6 is diagrams illustrated display screens displayed on an output unit of the jaundice meter according to the first embodiment.
FIG. 7 is diagrams illustrated other display screens displayed on the output unit of the jaundice meter according to the first embodiment.
FIG. 8 is a front perspective view illustrating a front appearance of a jaundice meter according to a second embodiment of the present invention.

### Description of Embodiments

With reference to the drawings, some embodiments of the present invention will now be described. In the figures, elements or components assigned with the same reference sign mean that they are the same elements or components, and their duplicated description will be appropriately omitted. In this specification, a collective term is designated by a reference sign without any suffix, and a term representing an individual element or component is designated by a reference sign with a suffix.

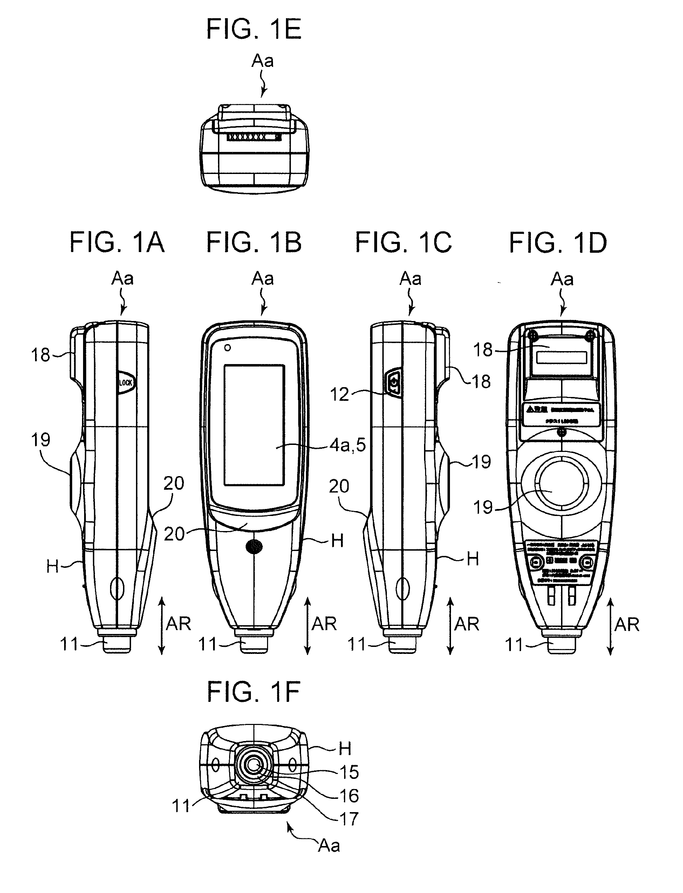

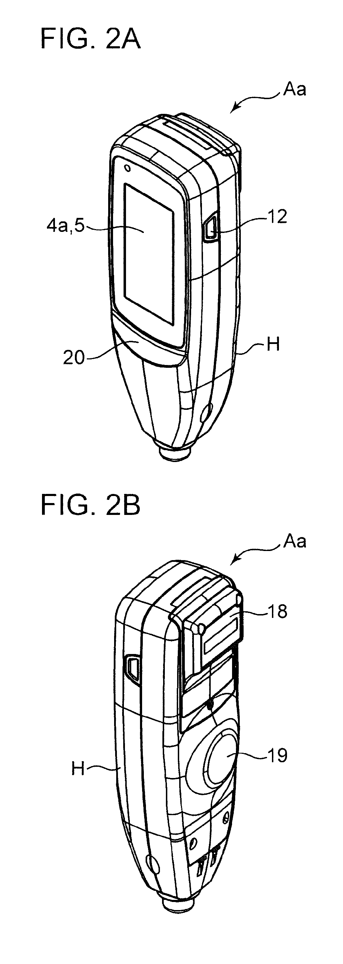

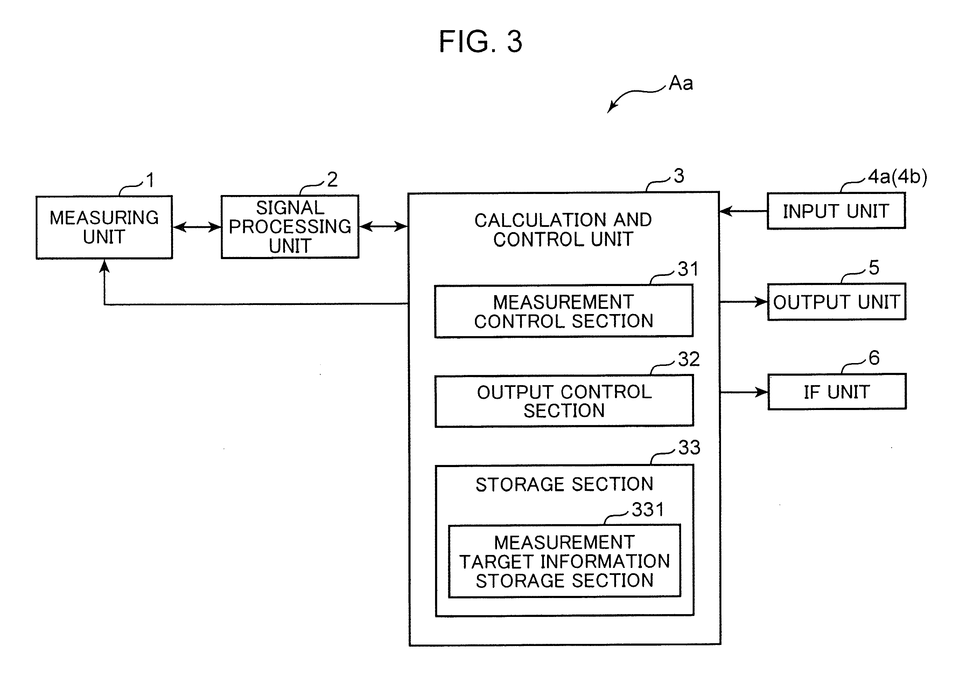

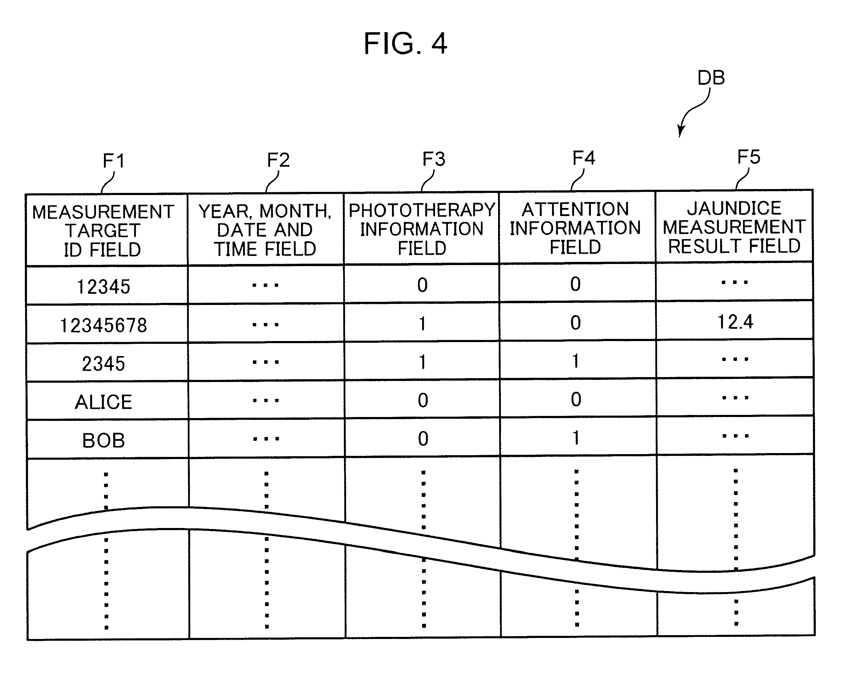

First of all, a configuration of a jaundice meter Aa will be described. FIG. 1 is six orthogonal views illustrating an external configuration of a jaundice meter according to a first embodiment. FIG. 1A, FIG. 1B, FIG. 1C, FIG. 1D, FIG. 1E and FIG. 1F are, respectively, a first side view (left side view), a front view, a second side view (right side view), a rear view, a base edge view, and a nose edge view when viewed from an entrance-exit port. FIG. 2 is perspective views illustrating the external configuration of the jaundice meter according to the first embodiment, wherein FIG. 2A is a perspective obverse view, and FIG. 2B is a perspective reverse view. FIG. 3 is a block diagram illustrating an electrical configuration of the jaundice meter according to the embodiment. FIG. 4 is an explanatory diagram of measurement target information stored in a measurement target information storage section of the jaundice meter according to the first embodiment.

The jaundice meter according to the embodiment is an optical apparatus designed to measure a degree of jaundice based on a phenomenon that a light component on a short wavelength side is absorbed by bilirubin deposited in a subcutaneous tissue in a greater amount as compared to a light component on a long wavelength side, wherein the jaundice meter comprises: a jaundice measurement unit configured to measure the degree of jaundice by using light; an input unit configured to allow therapeutic information of a measurement target to be input therethrough; a storage unit configured to store therein the therapeutic information input by using the input unit, and the degree of jaundice measured by the jaundice measurement unit, in mutually associated relation; and an output unit configured to, when outputting the degree of jaundice measured by the jaundice measurement unit, output the therapeutic information stored in the storage unit, together with the degree of jaundice.

More specifically, as illustrated in FIG. 1, the jaundice meter Aa is a handheld apparatus, and comprises a casing (housing, main frame) H having a size capable of being fitted into a hand of a user (a measurer, a medical staff such as a medical doctor or a nurse, or the like). The casing H is an approximately rectangular parallelepiped-shaped box-like member having one end (nose end) which is tapered toward an edge thereof. As illustrated in FIG. 1B, an aftermentioned input unit 4a and an aftermentioned output unit 5 are provided in a region of one principal surface (front surface) of the casing H on the side of the other end (in a base end region of the front surface). Further, a circular cylindrical-shaped protrusion-like probe 11 is provided on the side of the head end in such a manner as to be extendable and retractable with respect to the casing H, as indicated by the double arrow AR. The probe 11 is biased against the casing H in a protruding direction (a downward direction of the double arrow AR) by a non-illustrated biasing device (biasing member) such as a spring member, and is configured to be pushed into the casing H against a biasing force of the biasing device when a user presses the probe 11 perpendicularly onto a part (e.g., a forehead region or a chest region) of a living body of a person to be measured (measurement target), to thereby cause a light source of an aftermentioned measuring unit 1 to produce light. When the light source produces light, measurement light radiated from the light source exits an edge face of the probe 11, and enters a skin of the measurement target, and then backwardly scattered light in a subcutaneous tissue enters the aftermentioned measuring unit 1 inside the casing H, via the probe 11. That is, as illustrated in FIG. 1F, the edge face of the probe 11 is formed as an entrance-exit port serving as an exit port for emitting the measurement light therethrough, and an entrance port for introducing the scattered light therethrough. Further, as illustrated in FIG. 1C, a power on-off switch 12 for manually turning on and off the jaundice meter A is provided in a base end region of one side surface (right side surface) of the casing H. Further, as one device constituting the aftermentioned input unit 4a, a barcode reader 18 is provided in a region of the other principal surface (rear surface) of the casing H on the side of the other end (on a base end region of the rear surface), wherein it is configured to read a barcode, such as a one-dimensional barcode, or a two-dimensional barcode including an QR code (registered trademark), to thereby allow data represented by the barcode, to be input into the jaundice meter Aa therethrough.

A circular truncated cone-shaped direction setting convex portion 19 is provided on a region of the other principal surface including an approximately central position of the casing H, wherein it is configured to, when a user holds the casing H by his/her hand, set a direction of fingers of the user in such a manner as to allow a longitudinal direction of the fingers to intersect with the one direction of the casing H. Further, a thumb position setting portion 20 is provided on the one principal surface of the casing H, wherein it is configured to, when a user holds the casing H by his/her hand, set a position of a thumb of the user. More specifically, the casing H is formed such that a region thereof on the side of the nose end with respect to a display surface of the aftermentioned output unit 5 is raised higher than the display surface of the aftermentioned output unit 5 through an inclined surface (taper surface) or a concavely curved surface, and this inclined surface (taper surface) or the concavely curved surface serves as the thumb position setting portion 20. That is, a stepped portion having a difference in height with respect to the display surface is provided on the side of the nose end with respect to the display surface, and formed as an inclined surface (taper surface) or a concavely curved surface to serve as the thumb position setting portion 20. Then, the direction setting convex portion 19 is set on the basis of the thumb position setting portion 20. In this case, supposing a situation where a thumb of a user is located at a position set by the thumb position setting portion 20, the direction setting convex portion 19 is provided within a reachable region for two or more of the remaining first to fourth fingers. In the example illustrated in FIGS. 1 and 2, each of the direction setting convex portion 19 and the thumb position setting portion 20 is integrally formed with the casing H.

As illustrated in FIG. 3, the electrical configuration of the above optical jaundice meter Aa comprises a measuring unit 1, a signal processing unit 2, a calculation and control unit 3, an input unit 4a, an output unit 5, and an interface unit (hereinafter abbreviated as "IF unit") 6.

The measuring unit 1 is a device configured to, under control of the calculation and control unit 3, emit light to enter a skin, and measure two light components having different wavelengths in regard to backwardly scattered light which exits a surface of the skin as a result of scattering caused by a fatty layer or the like of a subcutaneous tissue having bilirubin deposited therein. For example, the measuring unit 1 comprises: a light source configured to produce measurement light; a first light guide member configured to guide the measurement light radiated from the light source, to the probe 11; second and third light guide members each configured to guide scattered light introduced into the probe 11; a first mirror configured to distribute light guided by the second light guide member into two light beams; a first light-receiving section configured to receive one of the two light beams distributed by the first mirror, through a first diffusing plate and a first optical filter; a second light-receiving section configured to receive a remaining one of the two light beams distributed by the first mirror, through a second diffusing plate and a second optical filter; a second mirror configured to distribute light guided by the third light guide member into two light beams; a third light-receiving section configured to receive one of the two light beams distributed by the second mirror, through a third diffusing plate and a third optical filter; and a fourth light-receiving section configured to receive a remaining one of the two light beams distributed by the second mirror, through a fourth diffusing plate and a fourth optical filter. The light source is composed, for example, of a columnar-shaped xenon lamp (xenon tube), and is capable of emitting, as measuring light, white light in the form of flash light. Each of the first to third light guide members is composed of an optical fiber bundle formed by bundling a plurality of optical fibers. An exit face 16 of the first light guide member for emitting the measurement light therethrough is formed in an annular shape (ring shape) to face outwardly at the edge face of the probe 11, as illustrated in FIG. 1F, and an entrance face 15 of the second light guide member for introducing the scattered light of the measurement light therethrough is formed in a circular shape at a position inward of the annular-shaped (ring-shaped) exit face 16 of the first light guide member to face outwardly at the edge face of the probe 11, as illustrated in FIG. 1F. Further, an entrance face 17 of the third light guide member for introducing the scattered light of the measurement light therethrough is formed in an annular shape (ring shape) at a position outward of the annular-shaped (ring-shaped) exit face 16 of the first light guide member to face outwardly at the edge face of the probe 11, as illustrated in FIG. 1F. That is, the exit face 16 of the first light guide member formed of a plurality of optical fibers, and the entrance faces 15, 17 of the second and third light guide members each formed of a plurality of optical fibers, are concentrically located. The exit face 16 and the entrance faces 15, 17 of the first to third light guide members are arranged in the above manner, so that the second light guide member can receive light propagated through a subcutaneous living tissue by a relatively long, first optical path length, and the third light guide member can receive light propagated through a subcutaneous living tissue by a relatively short, second optical path length. That is, the first optical path length is greater than the second optical path length (first optical path length > second optical path length). Each of the first and second mirrors is composed of a half mirror (semitransparent mirror) or a dichroic mirror. In the case where each of the first and second mirrors is composed of a dichroic mirror, for example, the dichroic mirror is configured to reflect light including a wavelength region of blue light, and transmit therethrough light including a wavelength region of green light. Each of the first to fourth optical filters is composed of a bandpass filter capable of filtering light to output a light component of a given transparent wavelength region. In this embodiment, each of the first and third optical filters is configured such that a center wavelength of the transparent wavelength region thereof becomes 450 nm so as to allow blue light to pass therethrough, and each of the second and fourth optical filters is configured such that a center wavelength of the transparent wavelength region thereof becomes 500 nm so as to allow green light to pass therethrough. Each of the first to fourth light-receiving sections is composed of a photoelectric conversion element capable of photoelectrically converting received-light to a current having a magnitude corresponding to an intensity of the received-light, e.g., a silicon photodiode. A detection result (received-light signals) obtained in the measuring unit 1 is output to the signal processing unit 2.

The signal processing unit 2 is a circuit designed to subject the detection result (received-light signals) obtained in the measuring unit 1 to a given signal processing, and output a result of the signal processing to the calculation and control unit 3. In order to subject the received-light signals from the first to fourth light-receiving sections to the signal processing, the signal processing unit 2 comprises first to fourth signal processing circuits each corresponding to a respective one of the first to fourth signal processing circuits. For example, each of the signal processing circuits comprises: an IV conversion section configured to convert each of the received-light signals from a current signal into a voltage signal; an amplification section configured to amplify an output of the IV conversion section at a predetermined given gain; and an AD conversion section configured to convert an output of the amplification section from an analog signal into a digital signal (to AD-convert an output of the amplification section). The AD conversion section is also configured to output a digital signal as a result of the conversion, to the calculation and control unit 3. That is, an output of the measuring unit 1 (the received-light signals from the first to fourth light-receiving sections) is input into the calculation and control unit 3 via the IV conversion sections, the amplification sections and the AD conversion sections.

The input unit 4a is a device designed to allow a variety of data associated with a jaundice measurement, e.g., various commands such as a command instructing the jaundice meter to start the jaundice measurement, and input data such as an identifier of a measurement target (measurement target ID), to be input into the jaundice meter Aa therethrough. The output unit 5 is a device designed to output a command and data input through the input unit 4a, and the degree of jaundice measured by the jaundice meter Aa, and composed, for example, of a display such as a CRT display, a liquid crystal display (LCD), an organic EL display or a plasma display, or a printing device such as a printer. In this embodiment, the input unit 4a comprises the aforementioned barcode reader 18, and a position input device, such as a resistive type or a capacitive type, configured to detect and input an operation position. In this embodiment, the output unit 5 is composed of a display, and a touch panel is made up of the position input device of the input unit 4a and the display of the output unit 5.

In this touch panel, the position input device is provided on a display surface of the display, and the display is configured to display thereon one or more candidates for inputtable contents, wherein when a user touches one position where a content that the user wants to input is displayed, the position is detected by the position input device, and the content displayed at the detected position is input into the jaundice meter Aa as a user's operation input content. In such a touch panel, a user is more likely to intuitively understand an input operation, so that it becomes possible to provide a jaundice meter Aa which is easy for a user to operate or handle. In addition, the display contents of the display can be changed to allow a user to input various contents so as to perform various operations to the jaundice meter Aa. In particular, a name, an identifier or the like of a person to be measured, as one example of the measurement target, can also be input, so that it becomes possible to provide a jaundice meter Aa compatible with electronic health records.

The IF unit 6 is a circuit designed to perform data input and output between an external device and the jaundice meter Aa, and composed, for example, of an interface circuit using IEEE 802.11 Standard Series, called Wi-Fi (wireless fidelity), or an interface circuit using Bluetooth (trademark) Standard, or an interface circuit configured to perform infrared communication using IrDA (Infrared Data Association) Standard or the like, or an interface circuit using USB (Universal Serial Bus) Standard.

The calculation and control unit 3 is designed to control each section of the jaundice meter Aa depending on a function of each section, so as to calculate the degree of jaundice. For example, the calculation and control unit 3 is composed of a micro-computer comprising: a CPU (Central Processing Unit); a non-volatile storage element, such as a RPM (Read Only Memory) or an EEPROM (Electrically Erasable Programmable Read Only Memory), preliminarily storing therein various programs to be executed by the CPU, data necessary for the execution and others; a volatile storage element, such as a RAM (Random Access Memory), serving as a so-called working memory for the CPU; and a peripheral circuit of the CPU. In the example illustrated in FIG. 3, the non-volatile storage element and the volatile storage element are depicted as a storage unit 33 having a measurement target information storage section 331. In the calculation and control unit 3, as a result of executing the programs, a measurement control section 31 and an output control section 32 are functionally formed.

The storage unit 33 is designed to store therein various programs, such as a jaundice calculation program for calculating the degree of jaundice from a detection result (received-light signals) in the measuring unit 1, a program for displaying data such as the degree of jaundice, and a program for controlling each section of the jaundice meter Aa, and various data, such as data necessary for executing the various data and data obtained by executing the various data. The storage unit 33 also serves as a working memory, as mentioned above.

The measurement target information storage section 331 of the storage unit 33 is designed to store therein measurement target information as various data regarding a measurement target. Examples of the measurement target information include measurement target ID as an identifier for specifying and identifying a measurement target, therapeutic information of the measurement target, and the degree of jaundice (jaundice measurement result) of the measurement target. The measurement target information storage section 331 is configured to store therein therapeutic information and jaundice measurement result of a measurement target, in mutually associated relation, and further store therein a measurement target ID of the measurement target, in associated relation with the therapeutic information and jaundice measurement result. More specifically, in this embodiment, for example, as illustrated in FIG. 4, the measurement target information is stored in the measurement target information storage section 331 as a measurement target information DB in tabular format. In FIG. 4, the measurement target information DB has a measurement target ID field F1, a date and clock time (year, month, date and time) field F2, a phototherapy information (information about the presence or absence of phototherapy) field F3, attention information (degree-of-attention information) field F4, and a jaundice measurement result field F5, and a record is created for each measurement target ID.

The measurement target ID field F1 is designed to allow a measurement target ID to be registered thereon. The date and clock time field F2 is designed to allow year, month, date and time when a measurement result is registered on the jaundice measurement result field F5, to be registered thereon. The phototherapy information field F3 is designed to allow information indicative of whether or not a phototherapy as a medical treatment for jaundice has been implemented for a measurement target (specifically, a newborn infant) identified by a measurement target ID registered on the measurement target ID field F1, to be registered thereon. In this embodiment, whether or not a phototherapy has been implemented is indicated, for example, by a flag, wherein a flag "1" indicates that the phototherapy has been implemented for the measurement target, and a flag "0" indicates that no phototherapy has been implemented for the measurement target. The attention information field F4 is designed to allow information indicative of how much attention should be given to a measurement target identified by a measurement target ID registered on the measurement target ID field F1, to be registered thereon. In this embodiment, the degree of attention is indicated, for example, by a flag, wherein a situation where the flag is set to "1" indicates that it is necessary to give greater attention to the measurement target than usual (high degree-of-attention), and a situation where the flag is set to "0" indicates that usual attention may be given to the measurement target (normal degree-of-attention). In this embodiment, as the degree of attention, there are two options: the normal degree-of-attention and the high degree-of-attention. Alternatively, the degree may be a plurality of degrees, for example, high, medium and normal degrees. The jaundice measurement result field F5 is designed to allow a jaundice measurement result indicative of the degree of jaundice in a measurement target identified by a measurement target ID registered on the measurement target ID field F1, to be registered thereon. The phototherapy information and attention information are one example of the aforementioned therapeutic information of the measurement target, and the jaundice measurement result is one example of the aforementioned jaundice measurement result (degree of jaundice) of the measurement target. As above, the measurement target information storage section 331 stores therein a measurement target ID, therapeutic information and jaundice measurement result of a measurement target in mutually associated relation, in tabular format.

The measurement control section 31 is designed to control the measuring unit 1 to thereby measure the degree of jaundice. The measuring unit 1 is operable, under control of the measurement control section 31, to emit white light as measurement light to enter a skin, and receive two light components having different wavelengths in regard to backwardly scattered light which exits a surface of the skin as a result of scattering caused by a fatty layer of a subcutaneous tissue, and then to output an obtained detection result (received-light signal) to the measurement control section 31 via the signal processing unit 2, as mentioned above. The measurement control section 31 is operable, based on the detection result, to calculate the degree of jaundice as a jaundice measurement result, and output the jaundice measurement result to the output unit 5 through the output control section 32. More specifically, the measurement control section 31 is operable, based on received-light signals indicative of light having a relatively long optical path length, detected by the first and second light-receiving sections, to calculate the degree of jaundice as a measurement result about light having a relatively long optical path length, and, based on received-light signals indicative of light having a relatively short optical path length, detected by the third and fourth light-receiving sections, to calculate the degree of jaundice as a measurement result about light having a relatively short optical path length, whereafter register, on the measurement target information DB, a jaundice measurement result calculated from the measurement result about light having a relatively long optical path length and the measurement result about light having a relatively short optical path length, to thereby store the jaundice measurement result in the measurement target information storage section 331, and output the jaundice measurement result to the output unit 5 through the output control section 32. The degree of jaundice is calculated from a difference between an intensity of blue light (center wavelength: 450 nm) and an intensity of green light (center wavelength: 550 nm) by the measurement control section 31. That is, the measurement control section 31 is operable to measure a degree of yellowness from the intensity of blue light on the basis of green light (a ratio of the intensity of blue light to the intensity of green light. For example, when the degree of yellowness becomes larger, the intensity of blue light on the basis of green light becomes lower, so that the degree of jaundice is determined to be relatively strong. On the other hand, when the degree of yellowness becomes smaller, the intensity of blue light on the basis of green light becomes higher, so that the degree of jaundice is determined to be relatively weak. That is, a degree of yellow coloring of bilirubin existing in a subcutaneous tissue of a living body is detected as a difference between optical densities in two wavelength regions of blue light and green light.

Then, in this embodiment, in order to register, on the date and clock time field F2, year, month, date and time when the jaundice measurement result is registered on the jaundice measurement result field F5, the measurement control section 31 is operable to measure year, month, date and time.

The output control section 32 is designed to control the output unit 5 to thereby control information (data) to be displayed on the output unit 5. More specifically, the output control section 32 is operable, when causing the degree of jaundice (jaundice measurement result) measured by the measuring unit 1, the signal processing unit 2 and the measurement control section 31, to be output to the output unit 5, to cause the therapeutic information stored in the measurement target information storage section 331, to be output to the output unit 5 together with the jaundice measurement result. The output control section 32 is also operable, when causing a jaundice measurement result of a measurement target to be output, after a measurement target ID of the measurement target is input through the input unit 4a, to cause therapeutic information stored in the measurement target information storage section 331 in associated relation with the measurement target ID, to be output to the output unit 5 together with the jaundice measurement result. In this case, the output control section 32 may be configured to cause a measurement target ID stored in the measurement target information storage section 331 and therapeutic information stored in the measurement target information storage section 331 in associated relation with the measurement target ID, to be output to the output unit 5 in the form of a medical record. The output control section 32 may also be configured to cause a measurement target ID stored in the measurement target information storage section 331 and therapeutic information stored in the measurement target information storage section 331 in associated relation with the measurement target ID, to be output to the output unit 5 in the form of a list for a plurality of different measurement target IDs.

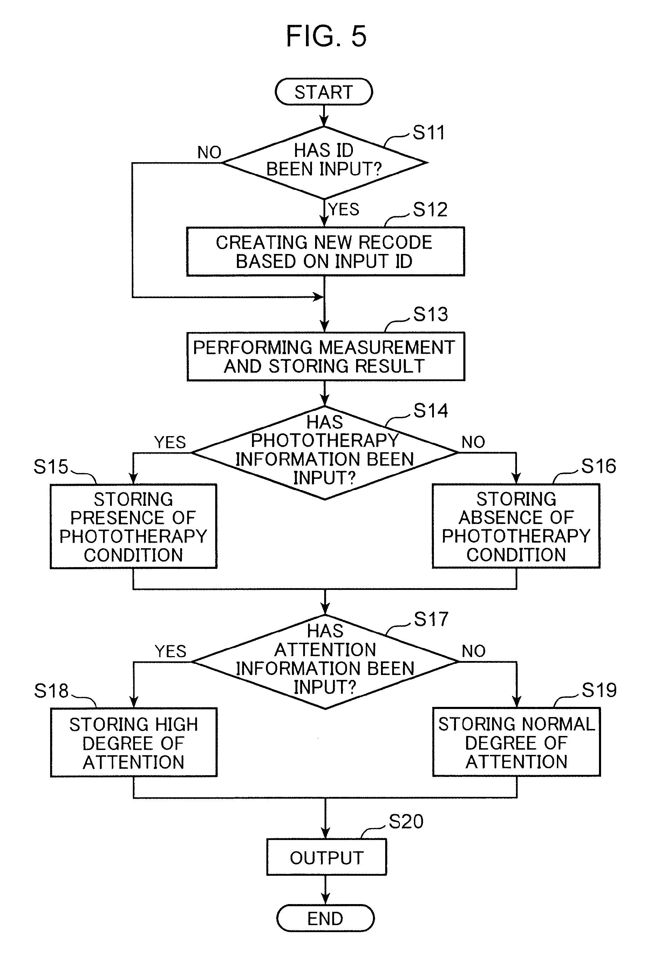

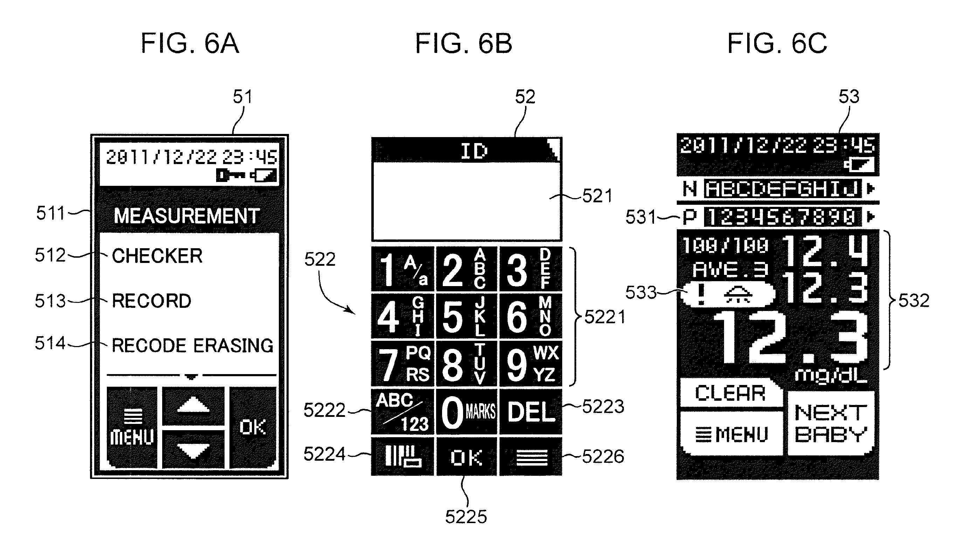

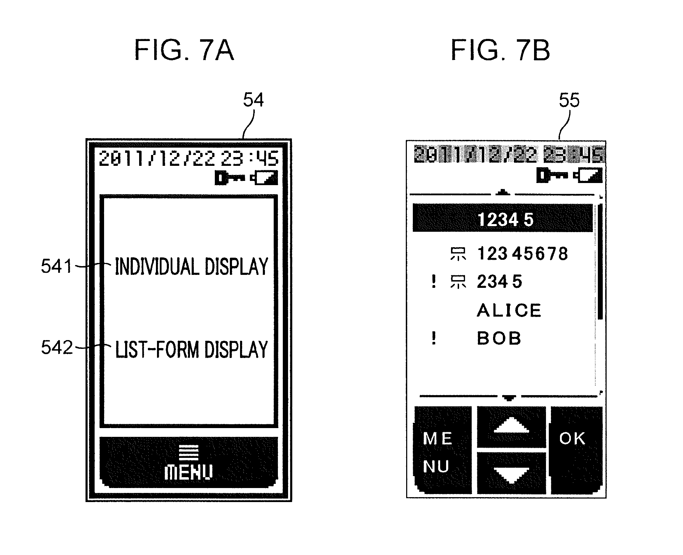

Next, an operation of the jaundice meter Aa configured as above will be described. FIG. 5 is a flowchart illustrating an operation of the jaundice meter according to the first embodiment. FIG. 6 is diagrams illustrated display screens displayed on the output unit of the jaundice meter according to the first embodiment. FIG. 6A, FIG. 6B and FIG. 6C illustrate a main menu input screen, a measurement target ID input screen, and a jaundice measurement result display screen, respectively. FIG. 7 is diagrams illustrated other display screens displayed on the output unit of the jaundice meter according to the first embodiment. FIG. 7A illustrates a selective input screen for selecting one of individual display and list-form display, and FIG. 7B illustrates a list-form display screen.

When the power on-off switch 12 of the jaundice meter Aa is manually operated to activate the jaundice meter Aa, the output control section 32 of the calculation and control unit 3 operates to cause a main menu input screen for inputting, into the jaundice meter Aa, an instruction for an operation mode of the jaundice meter Aa so as to operate the jaundice meter Aa in the operation mode, to be output to the output unit 5. For example, in this embodiment, a main menu input screen 51 illustrated in FIG. 6A is displayed on a portion of the output unit 5 constituting the touch panel. This main menu input screen 51 has: a "measurement" field 511 for inputting, into the jaundice meter Aa, an instruction for measurement of the degree of jaundice so as to operate the jaundice meter Aa in a measurement mode during which the degree of jaundice is measured; a "checker" field 512 for inputting, into the jaundice meter Aa, an instruction for execution of inspection of the jaundice meter Aa so as to operate the jaundice meter Aa in an inspection mode during which the inspection is performed; a "record" field 513 for inputting, into the jaundice meter Aa, an instruction for display of past jaundice measurement results so as to operate the jaundice meter Aa in a record mode during which the past jaundice measurement results are displayed; and a "record erasing" field 514 for inputting, into the jaundice meter Aa, an instruction for erasing of past jaundice measurement results so as to operate the jaundice meter Aa in a record erasing mode during which the past jaundice measurement results are erased. A user touches a display area of the "measurement" field 511 in the main menu input screen displayed on the output unit 5 of the touch panel to thereby input an instruction for starting the measurement, into the jaundice meter Aa.

In FIG. 5, upon input of the instruction on start of the measurement into the jaundice meter Aa, the measurement control section 31 of the calculation and control unit 3 instructs the output control section 32 to cause a measurement target ID input screen for inputting a measurement target ID therethrough, to be output to the output unit 5, and determines whether or not a measurement target ID has been input through the touch panel composed of the input unit 4 and the output unit 5 (S11). In this embodiment, for example, as illustrated in FIG. 6B, the measurement target ID input screen 52 has a measurement target ID display field 521 in which a character (including alphabetical characters, numeric characters and marks) input as a measurement target ID is displayed, and a keyboard display field 522 in which a keyboard for selectively input a character is displayed. The keyboard display field 522 has: a numerical key set 5221 consisting of numerical keys "0" to "9"; a switching key 5222 for switching between an alphabet input mode for imputing alphabetical characters and a numeral input mode for inputting numeric characters; a delete key 5223 for deleting input characters one-by-one in order from a lastly input character; a barcode read key 5224 for reading a barcode by the barcode reader 18; a confirmation input key (Enter key) 5225 for confirming input of a character string displayed on the measurement target ID display field 521; and a main menu switching key 5226 for switching the screen display on the output unit 5 from the measurement target ID input screen to the main menu input screen. The measurement target ID input screen 52 is configured such that the numerical keys "2" to "9" of the numerical key set 5221 are additionally assigned with alphabetical characters (alphabets), and can also be used to input alphabets by the switching operation of the switching key 5222. Alternatively, a numeral input screen for inputting numeric characters and an alphabet input screen for inputting alphabetical characters may be configured as separate screens. Further, the measurement target ID may be input, for example, by reading a barcode on a wristband attached to a measurement target, using the barcode reader 18, or by additionally providing an RF ID reader to the jaundice meter Aa, and reading an RF ID attached to a measurement target, using the RF ID reader.

When it is determined that no measurement target ID has not been input (NO), for example, when the confirmation input key 5225 is touched (NO) in a situation where no data is input into the measurement target ID input screen 52 (no data is displayed on the measurement target ID display field 521), the measurement control section 31 performs processing S 13. That is, the measurement control section 31 operates to perform measurement without any measurement target ID, and store a jaundice measurement result in the storage unit 33.

On the other hand, when it is determined that a measurement target ID has been input (YES), the measurement control section 31 performs the processing S13 after performing processing S12. In the processing S12, the measurement control section 31 operates to ensure a storage region for storing measurement target information associated with the input measurement target ID, in the storage unit 33. More specifically, the measurement control section 31 creates a new record in the measurement target information DB, based on the input measurement target ID. In this way, a storage region for storing measurement target information associated with the input measurement target ID is ensured in the measurement target information storage section 331.

Then, in the processing S13 after a measurement target ID has been input, the measurement control section 31 operates to perform the measurement and store an obtained jaundice measurement result in the storage unit 33 in associated relation with the measurement target ID. More specifically, the measurement control section 31 registers current year, month, date and time on the date and clock time field F2, and registers the jaundice measurement result on the jaundice measurement result field F5, in the record newly created in the processing S12.

Then, the measurement control section 31 instructs the output control section 32 to cause a phototherapy information input screen for inputting phototherapy information, to be output to the output unit 5, and determines whether or not the phototherapy information has been input through the input unit 4a (S14). For example, the phototherapy information input screen has a "YES" field for inputting information indicating that the phototherapy has been implemented (presence of phototherapy), and a "NO" field for inputting information indicating that no phototherapy has been implemented (absence of phototherapy).

When the "YES" field on the phototherapy information input screen is touched, the measurement control section 31 determines, in the processing S 14, that the presence of phototherapy has been selected and input (YES), and performs processing S 17 after performing processing S15. In the processing S 15, the measurement control section 31 stores information indicative of the presence of phototherapy, in the storage unit 33 in associated relation the measurement target ID. More specifically, the measurement control section 31 registers a flag "1" indicative of the presence of phototherapy, on the phototherapy information field F3 in the record newly created in the processing S12.

On the other hand, when the "NO" field on the phototherapy information input screen is touched, the measurement control section 31 determines, in the above determination step, that the absence of phototherapy has been selected and input (NO), and performs processing S17 after performing processing S16. In the processing S16, the measurement control section 31 stores information indicative of the absence of phototherapy, in the storage unit 33 in associated relation the measurement target ID. More specifically, the measurement control section 31 registers a flag "0" indicative of the absence of phototherapy, on the phototherapy information field F3 in the record newly created in the processing S12.

Then, in the processing S 17, the measurement control section 31 instructs the output control section 32 to cause a degree-of-attention information input screen for inputting attention information, to be output to the output unit 5, and determines whether or not the degree-of-attention information has been input through the input unit 4a. For example, the degree-of-attention information input screen has a "YES" field for inputting information indicative of the high degree-of-attention, and a "NO" field for inputting information indicative of the normal degree-of-attention.

When the "YES" field on the attention information input screen is touched, the measurement control section 31 determines, in the processing S 17, that the high degree-of-attention has been selected and input (YES), and performs processing S20 after performing processing S18. In the processing S18, the measurement control section 31 stores information indicative of the high degree-of-attention, in the storage unit 33 in associated relation the measurement target ID. More specifically, the measurement control section 31 registers a flag "1" indicative of the high degree-of-attention, on the attention information field F4 in the record newly created in the processing S 12.

On the other hand, when the "NO" field on the attention information input screen is touched, the measurement control section 31 determines, in the processing S 17, that the normal degree-of-attention has been selected and input (NO), and performs the processing S20 after performing processing S 19. In the processing S 19, the measurement control section 31 stores information indicative of the normal degree-of-attention, in the storage unit 33 in associated relation the measurement target ID. More specifically, the measurement control section 31 registers a flag "0" indicative of the normal degree-of-attention, on the attention information field F4 in the recode newly created in the processing S12.

As above, in the processing S14 or the processing S19, the phototherapy information and the attention information are input and stored in the storage unit 33 individually and sequentially. Alternatively, such storage operations may be collectively performed in a therapeutic information input screen for inputting therapeutic information such as the phototherapy information and the attention information. This therapeutic information input screen has: a "---, ---" field for inputting the normal degree-of-attention and the absence of phototherapy; a "!, ---" field for inputting the high degree-of-attention and the absence of phototherapy; a "---, ∩∩" field for inputting the normal degree-of-attention and the presence of phototherapy; and a "!, ∩∩" field for inputting the high degree-of-attention and the presence of phototherapy. When the "---, ---" field is touched in this screen, the measurement control section 31 determines that the normal degree-of-attention and the absence of phototherapy are selected and input, and stores information indicative of the normal degree-of-attention and the absence of phototherapy in the storage unit 33 in associated relation with the measurement target ID. When the "!, ---" field is touched in this screen, the measurement control section 31 determines that the high degree-of-attention and the absence of phototherapy are selected and input, and stores information indicative of the high degree-of-attention and the absence of phototherapy in the storage unit 33 in associated relation with the measurement target ID. When the "---, ∩∩" field is touched in this screen, the measurement control section 31 determines that the normal degree-of-attention and the presence of phototherapy are selected and input, and stores information indicative of the normal degree-of-attention and the presence of phototherapy in the storage unit 33 in associated relation with the measurement target ID. When the "!, ∩∩" field is touched in this screen, the measurement control section 31 determines that the high degree-of-attention and the presence of phototherapy are selected and input, and stores information indicative of the high degree-of-attention and the presence of phototherapy in the storage unit 33 in associated relation with the measurement target ID.

Then, in the processing S20, the measurement control section 31 instructs the output control section 32 to cause a measurement result display screen for outputting a jaundice measurement result, to be output to the output unit 5. In this case, the output control section 32 operates to cause the therapeutic information stored in the measurement target information storage section 331, to be output to the output unit 5 together with the jaundice measurement result. Alternatively, the output control section 32 operates to cause the measurement target ID and the therapeutic information stored in the measurement target information storage section 331, to be output to the output unit 5 together with the jaundice measurement result.

For example, in this embodiment, as illustrated in FIG. 6C, the measurement result display screen 53 has: a measurement target ID display field 531 for displaying a measurement target ID; a measurement result display field 532 for displaying a jaundice measurement result; and a therapeutic information display field 533 for displaying therapeutic information (presence or absence of phototherapy, and degree of attention). Through this screen, the jaundice measurement result is output to the output unit 5 together with the measurement target ID and the therapeutic information.

In the jaundice meter Aa according to this embodiment, based on above operation, a measurement target ID and therapeutic information are input through the input unit 4a, and the measurement target ID, the therapeutic information and a jaundice measurement result are stored in the measurement target information storage section 331 of the storage unit 33 in mutually associated relation. Then, when the jaundice measurement result is output, the measurement target ID and the therapeutic information stored in the measurement target information storage section 331 are also output together with the jaundice measurement result.

In the jaundice meter Aa according to this embodiment, when a user wants to output, to the output unit 5, the therapeutic information stored in the measurement target information storage section 331 of the storage unit 33, the jaundice meter Aa operates as follows.

First of all, a user operates the jaundice meter Aa to display the main menu (main menu input screen) 51 on the output unit 5, and touches the "record" field 513 in the main menu 51 displayed on the output unit 5 to thereby input of an instruction on record display into the jaundice meter Aa.

Upon input of the instruction on record display into the jaundice meter Aa, the output control section 32 operates to cause the selective input screen (individual/list-form selective input screen) 54 for selecting whether the therapeutic information is displayed individually for a specific measurement target ID, or in the form of a list for a plurality of measurement target IDs, and determines which of the display formats is selected and input through the touch panel. For example, in this embodiment, as illustrated in FIG. 7A, the selective input screen 54 has an "individual display" field 541 for selecting and inputting individual display for a specific measurement target ID, and a "list-form display" field 542 for selecting and inputting list-form display for a plurality of measurement target IDs.

When selecting the individual display, the user touches the "individual display" field 541 in the selective input screen 54 displayed on the output unit 5 to thereby input an instruction on the individual display into the jaundice meter Aa. Upon input of the instruction on the individual display into the jaundice meter Aa, the output control section 32 operates to cause a screen similar, for example, to the ID input screen (measurement target ID input screen) 52 illustrated in FIG. 6B, to be display on the output unit 5 to thereby prompt the user to input a measurement target ID of the specific measurement target pertaining to the individual display. When the measurement target ID is input, the output control section 32 retrieves therapeutic information associated with the measurement target ID input through the input unit 4a, from the measurement target information DB of the measurement target information storage section 331, and operates to cause the retrieved therapeutic information to be displayed on the output unit 5. The jaundice meter Aa may be configured to display the jaundice measurement result on the output unit 5, in addition to the therapeutic information. In this case, the user can refer to the therapeutic information in combination with the jaundice measurement result. Further, the jaundice meter Aa may be configured to display most recently input therapeutic information on the output unit 5 without seeking input of a measurement target ID. This makes it possible to simplify the operation when the jaundice meter Aa is exclusively used for one measurement target.

On the other hand, when selecting the list-form display, the user touches the "list-form display" field 542 in the selective input screen 54 displayed on the output unit 5 to thereby input an instruction on the list-form display into the jaundice meter Aa. Upon input of the instruction on the list-form display into the jaundice meter Aa, the output control section 32 operates to cause the therapy display screen (list-form therapeutic information display screen) 55 for displaying therapeutic information in the form of a list, to be displayed on the output unit 5. For example, in this embodiment, as illustrated in FIG. 7B, attention information, phototherapy information and measurement target information ID are display on one row in this order, and such a row (phototherapy information display row) is displayed on the therapy display screen 55, in plural number. That is, a plurality of sets of therapeutic information and phototherapy information as attention information are displayed in associated relation with a plurality of measurement target IDs, respectively. In the embodiment illustrated in FIG. 7B, each of the attention information and the phototherapy information is indicated by the present or absence of a given symbol. A displayed state of the aforementioned symbol at a display position of the attention information means that the measurement target requires a high degree-of-attention. On the other hand, an non-displayed state of the aforementioned symbol at the display position of the attention information means that the measurement target requires only a normal degree-of-attention. A displayed state of the aforementioned symbol at a display position of the phototherapy information means the presence of phototherapy. On the other hand, a non-display state of the aforementioned symbol at the display position of the phototherapy information means the absence of phototherapy. The therapy display screen 55 has scroll keys. A user can operates the scroll keys to change therapeutic information to be displayed on the therapy display screen 55 in units of row, so as to display therapeutic information associated with another measurement target ID.

As regards therapeutic information to be displayed on the therapy display screen 55, the jaundice meter Aa may be configured to select and display give rows of most recently input therapeutic information. Alternatively, the jaundice meter Aa may be configured to display therapeutic information associated with designated measurement target IDs. In this case, the output control section 32 operates to cause an ID input screen similar, for example, to the measurement target ID input screen 52 illustrated in FIG. 6B to be displayed on the output unit 5 to thereby prompt a user to input measurement target IDs for list-form display. When the measurement target IDs are input, the output control section 32 retrieves therapeutic information associated with the measurement target IDs input through the input unit 4a, from the measurement target information DB of the measurement target information storage section 331, and operates to cause the retrieved therapeutic information to be displayed on the output unit 5. The jaundice meter Aa may be configured to display jaundice measurement results on the output unit 5, in addition to the therapeutic information.

As described above, in the jaundice meter Aa acceding to the first embodiment, therapeutic information is input through the input unit 4a, and the therapeutic information and a jaundice measurement result (degree of jaundice) measured in the above manner are stored in the measurement target information storage section 331 of the storage unit 33 in mutually associated relation. Then, when outputting the measured jaundice measurement result, the therapeutic information stored in the measurement target information storage section 331 is output to the output unit 5 together with the jaundice measurement result. Therefore, the jaundice meter Aa acceding to the first embodiment can output therapeutic information regarding a medical treatment for a measurement target. In addition, the jaundice meter Aa acceding to the first embodiment allows a user to refer to an actually measured jaundice measurement result, while taking into account of the therapeutic information, and adequately make a judgment on the degree of jaundice. Particularly, in case of the presence of phototherapy, a warning range in a measurement value of the jaundice measurement result changes. Thus, the display of therapeutic information is useful. In the jaundice meter Aa configured as above, therapeutic information is stored in the measurement target information storage section 331, so that it becomes possible to check therapeutic information by a single device without a need for checking therapeutic information by a medical record or the like.

The jaundice meter Aa acceding to the first embodiment can output information about the presence or absence of phototherapy as one therapeutic information to allow a user to check the presence or absence of phototherapy.

The jaundice meter Aa acceding to the first embodiment can also output attention information indicative of a degree of attention required for a patient, as one therapeutic information. This makes it possible to manage a patient as a measurement target, depending on a symptomatic state, by using a single jaundice meter Aa, and delicately take care of the patient. In addition, even when a person responsible for taking care of the patient as the measurement target is changed, a new person can take over the attention information of the patient by referring to the jaundice meter Aa.

In the jaundice meter Aa acceding to the first embodiment, therapeutic information stored in the measurement target information storage section 331 of the storage unit 33 can be managed by respective measurement target IDs, so that therapeutic information can be managed with respect to a plurality of measurement targets. This makes it possible to use a single jaundice meter Aa for a plurality of measurement targets while managing therapeutic information on a per-measurement target basis. Further, it becomes possible to organize jaundice measurement results on a per-measurement target ID basis, and figure out a tendency of jaundice measurement results on a per-measurement target ID basis.

The jaundice meter Aa acceding to the first embodiment can output a measurement target ID and therapeutic information to the output unit in the form of a record, so that it becomes possible to check therapeutic information of a measurement target.

The jaundice meter Aa acceding to the first embodiment can output information in the form of a list, so that it becomes possible to refer to a plurality of therapeutic information for a plurality of measurement targets, at once.

The jaundice meter Aa acceding to the first embodiment comprises the IF unit 6, so that it becomes possible to output information to an external device. Thus, in the jaundice meter Aa acceding to the first embodiment, therapeutic information of a measurement target can be referred to by using an external device different from the jaundice meter Aa, and information stored in the storage unit 33 can be handled by using the external device, so that processing and storing of information becomes relatively easy, for example. Such a jaundice meter Aa is compatible with an electronic health record.

Next, a second embodiment of the present invention will be described.

### (Second Embodiment)

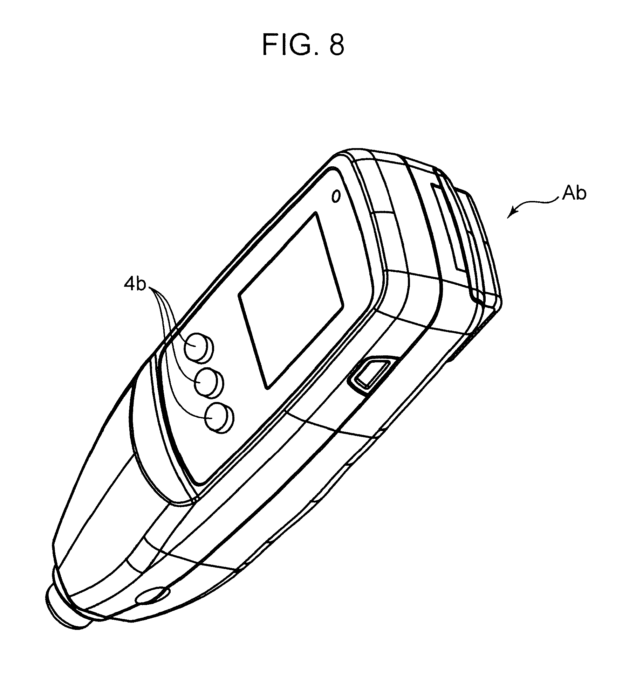

FIG. 8 is a front perspective view illustrating a front appearance of a jaundice meter according to a second embodiment of the present invention. In the jaundice meter Aa according to the first embodiment, the touch panel position input device 4a is employed as one example of the input unit. In a jaundice meter Ab according to the second embodiment, in place of or in addition to the touch panel position input device 4a, as another example of an input unit 4b, a plurality of mechanical switches 4b are employed. Thus, the jaundice meter Ab according to the second embodiment is the same as the jaundice meter Aa according to the first embodiment, except for the input unit 4b, and therefore description of the common structure will be omitted.

As illustrated in FIG. 8, the input unit 4b in the jaundice meter Ab according to the second embodiment comprises a plurality of push-button switches 4b. Each of the plurality of push-button switches 4b is disposed between the display screen of the output unit 5 and the thumb position setting portion 20, and configured to open and close a switch according to a push movement. Each of the push-button switches 4b is associated with a predetermined given input content, and, when the push-button switch is pushed, the input content is input into the jaundice meter Aa. In this way, a plurality of switches 4b may be employed as the input unit 4.

While this specification discloses various techniques as mentioned above, major ones of the techniques will be outlined below.

According to one aspect of the present invention, there is provided a jaundice meter which comprises: a jaundice measurement unit configured to measure a degree of jaundice by using light; an input unit configured to allow therapeutic information regarding a medical treatment for a measurement target to be input therethrough; a storage unit configured to store therein the therapeutic information input through the input unit, and the degree of jaundice measured by the jaundice measurement unit, in mutually associated relation; and an output unit configured to, when outputting the degree of jaundice measured by the jaundice measurement unit, output the therapeutic information stored in the storage unit, together with the degree of jaundice.

In the jaundice meter of the present invention, therapeutic information is input through the input unit, and the therapeutic information and the degree of jaundice measured by the jaundice measurement unit are stored in the storage unit in mutually associated relation. Then, when outputting the degree of jaundice measured by the jaundice measurement unit, the output unit outputs the therapeutic information stored in the storage unit together with the degree of jaundice. Therefore, the jaundice meter can output therapeutic information regarding a medical treatment for a measurement target. In the jaundice meter, therapeutic information is stored in the storage unit, so that it becomes possible to check therapeutic information by a single device without a need for checking therapeutic information by a medical record or the like.

According to one specific embodiment, in the jaundice meter of the present invention, the therapeutic information includes phototherapy information indicative of whether or not a phototherapy as a medical treatment for jaundice symptoms has been implemented for the measurement target.

The jaundice meter having this feature can output information about the presence or absence of phototherapy as therapeutic information to allow a user to check the presence or absence of phototherapy.

According to another specific embodiment, in the above jaundice meter, the therapeutic information includes attention information indicative of how much attention should be paid to the measurement target.

The jaundice meter having this feature can output degree-of-attention information indicative as therapeutic information. This makes it possible to manage a patient as the measurement target, depending on a symptomatic state, by using a single jaundice meter, and delicately take care of the patient. In addition, even when a person responsible for taking care of the patient as the measurement target is changed, a new person can take over the degree-of-attention information of the patient by referring to the jaundice meter.

According to another specific embodiment, in the above jaundice meter, the output unit comprises a display configured to display the therapeutic information on a screen.

This makes it possible to provide a jaundice meter capable of checking therapeutic information by means of image display.

According to another specific embodiment, in the above jaundice meter, the input unit comprises an identifier input section configured to allow an identifier for specifying and identifying the measurement target to be input therethrough, and wherein the storage unit is configured to store the identifier of the measurement target input through the identifier input section, in associated relation to the therapeutic information and the degree of jaundice, and the output unit is configured to, when outputting the degree of jaundice measured by the jaundice measurement unit, output the identifier of the measurement target input through the identifier input section, and the therapeutic information stored in the storage unit in associated relation with the identifier of the measurement target, together with the degree of jaundice.

In the jaundice meter having this feature, therapeutic information stored in the storage unit can be managed by respective identifiers, so that therapeutic information can be managed with respect to a plurality of measurement targets. This makes it possible to use a single jaundice meter for a plurality of measurement targets while managing therapeutic information on a per-measurement target basis. Further, it becomes possible to organize the degree of jaundice on a per-measurement target basis, and figure out a tendency of the degree of jaundice on a per-measurement target basis.

According to another specific embodiment, the above jaundice meter further comprises an output control section configured to cause an identifier of the measurement target stored in the storage unit, and the therapeutic information stored in the storage unit in associated relation with the identifier of the measurement target, to be output to the output unit.

The jaundice meter having this feature can output an identifier of a measurement target and therapeutic information to the output unit, so that it becomes possible to check therapeutic information of a measurement target.

According to another specific embodiment, in the above jaundice meter, the output control section is configured to cause the identifier of the measurement target stored in the storage unit, and the therapeutic information stored in the storage unit in associated relation with the identifier of the measurement target, to be output to the output unit in the form of a list for a plurality of different identifiers.

The jaundice meter having this feature can output information in the form of a list, so that it becomes possible to refer to a plurality of therapeutic information for a plurality of measurement targets, at once.

According to another specific embodiment, in the above jaundice meter, wherein the output unit comprises an interface unit configured to allow information to be output to an external device therethrough.

The jaundice meter having this feature comprises the interface unit, so that it becomes possible to output information to the external device. Thus, in the jaundice meter having the above feature , therapeutic information of a measurement target can be referred to by using an external device different from the jaundice meter, and information stored in the storage unit can be handled by using the external device, so that processing and storing of information becomes relatively easy, for example. Such a jaundice meter is compatible with an electronic health record.

According to another aspect of the present invention, there is provided an output method for a jaundice meter, which comprises: a jaundice measurement step of measuring a degree of jaundice by using light; an input step of inputting therapeutic information regarding a medical treatment for a measurement target; a storage step of storing the therapeutic information input in the input step, and the degree of jaundice measured in the jaundice measurement step, in associated relation with each other; and an output step of, when outputting the degree of jaundice measured in the jaundice measurement step, outputting the therapeutic information stored in the storage unit in the storage step, together with the degree of jaundice.

In the output method of the present invention, therapeutic information is input in the input step, and the therapeutic information and the degree of jaundice measured in the jaundice measurement step are stored in the storage unit in mutually associated relation. Then, when outputting the degree of jaundice measured in the jaundice measurement step, the output unit outputs the therapeutic information stored in the storage unit together with the degree of jaundice. Therefore, the above output method can output therapeutic information regarding a medical treatment for a measurement target. In the above output method, therapeutic information is stored in the storage unit, so that it becomes possible to check therapeutic information by a single device without a need for checking therapeutic information by a medical record or the like.

This application is based on Japanese Patent Application Serial No. 2012-148418 filed in Japan Patent Office on July 02, 2012, the contents of which are hereby incorporated by reference.

Although the present invention has been adequately and fully described by way of example with reference to the accompanying drawings, it is to be understood that various changes and/or modifications will be apparent to those skilled in the art. Therefore, unless otherwise such changes and modifications depart from the scope of the present invention hereinafter defined, they should be construed as being included therein.

### Industrial Applicability

The present invention can provide a jaundice meter and an output method for the jaundice meter.

## Claims

1. A jaundice meter comprising:
a jaundice measurement unit configured to measure a degree of jaundice by using light;
an input unit configured to allow therapeutic information regarding a medical treatment for a measurement target to be input therethrough;
a storage unit configured to store therein the therapeutic information input through the input unit, and the degree of jaundice measured by the jaundice measurement unit, in mutually associated relation; and
an output unit configured to, when outputting the degree of jaundice measured by the jaundice measurement unit, output the therapeutic information stored in the storage unit, together with the degree of jaundice.

2. The jaundice meter as defined in claim 1, wherein the therapeutic information includes phototherapy information indicative of whether or not a phototherapy as a medical treatment for jaundice symptoms has been implemented for the measurement target.

3. The jaundice meter as defined in claim 1 or 2, wherein the therapeutic information includes attention information indicative of how much attention should be paid to the measurement target.

4. The jaundice meter as defined in any one of claims 1 to 3, wherein the output unit comprises a display configured to display the therapeutic information on a screen.

5. The jaundice meter as defined in any one of claims 1 to 4, wherein the input unit comprises an identifier input section configured to allow an identifier for specifying and identifying the measurement target to be input therethrough, and wherein
the storage unit is configured to store the identifier of the measurement target input through the identifier input section, in associated relation to the therapeutic information and the degree of jaundice, and
the output unit is configured to, when outputting the degree of jaundice measured by the jaundice measurement unit, output the identifier of the measurement target input through the identifier input section, and the therapeutic information stored in the storage unit in associated relation with the identifier of the measurement target, together with the degree of jaundice.

6. The jaundice meter as defined in any one of claims 1 to 5, which further comprises an output control section configured to cause an identifier of the measurement target stored in the storage unit, and the therapeutic information stored in the storage unit in associated relation with the identifier of the measurement target, to be output to the output unit.

7. The jaundice meter as defined in claim 6, wherein the output control section is configured to cause the identifier of the measurement target stored in the storage unit, and the therapeutic information stored in the storage unit in associated relation with the identifier of the measurement target, to be output to the output unit in the form of a list for a plurality of different identifiers.

8. The jaundice meter as defined in any one of claims 1 to 7, wherein the output unit comprises an interface unit configured to allow information to be output to an external device therethrough.

9. An output method for a jaundice meter, comprising:
a jaundice measurement step of measuring a degree of jaundice by using light;
an input step of inputting therapeutic information regarding a medical treatment for a measurement target;
a storage step of storing the therapeutic information input in the input step, and the degree of jaundice measured in the jaundice measurement step, in associated relation with each other; and
an output step of, when outputting the degree of jaundice measured in the jaundice measurement step, outputting the therapeutic information stored in the storage unit in the storage step, together with the degree of jaundice.
